# EUROPEAN PATENT APPLICATION

(11) **EP 4 714 486 A1**
(43) Date of publication of application: **25.03.2026**
(21) Application number: 25203435.0
(22) Date of filing: 19.09.2025
(51) Int. Cl.: A61M 25/00, A61F 2/95, A61M 25/01, A61M 29/00, A61M 25/06

(54) **MEDICAL DELIVERY SYSTEM**

(30) Priority: 19.09.2024 CN 202411313006
(71) Applicant: Hangzhou Valgen Medtech Co., Ltd., Hangzhou, Zhejiang 310051 (CN)
(72) Inventor: Zhang, Tingchao, Zhejiang, 310051 (CN); Yuan, Yongxin, Zhejiang, 310051 (CN); Li, Liguang, Zhejiang, 310051 (CN)
(74) Representative: Berggren Oy

(57) **Abstract**

The present invention provides a medical delivery system (100), comprising a first delivery device (20), a second delivery device (30) and at least one limiting member (40). The first delivery device has a first catheter (21) and a first handle (22), and the second delivery device has a second catheter (31) and a second handle (32). The second catheter is extended through the first delivery device, and the second handle is positioned at a proximal side of the first handle. Each of the limiting members is removably coupled to a proximal end portion of the second catheter and is axially limited between the first handle and the second handle, so as to limit each of at least one axial displacement of the second handle relative to the first handle in a one-to-one correspondence, thereby controlling each of at least one axial displacement of the second catheter relative to the first catheter in a one-to-one correspondence.

## Description

### Technical Field

The present invention relates to the technical field of medical instruments, and in particular to a medical delivery system.

### Background

Heart disease is one of major health threats around the world, among which heart valve diseases are the most common. Treatment with interventional surgery for heart valve diseases caused by mitral valve diseases or tricuspid valve diseases has become increasingly common, and it needs to rely on Rays and Ultrasound to provide a guidance to reach a diseased region of a patient through transcatheter techniques. Relatively to the ray guidance, the ultrasound guidance is safe for the patient, but requires rich imaging experience for an operator. Especially in TEER surgery with transfemoral approach, since operation steps are cumbersome and the learning curve is long, the ultrasound guidance still needs to rely on the ray guidance together for navigation and monitoring. However, long-term ray exposure is easy to cause radiation damage to the operator and the patient, which is danger to life and health for the operator and the patient.

### Summary

An object of the present invention is to provide a medical delivery system, which ensures reliability under ultrasound guidance, and reduces dependence on ray guidance, thereby greatly protecting the life and health of users and patients, and effectively improving the safety of interventional surgery.

To achieve the described object, some embodiments of the present invention provide a medical delivery system having a limiting function, including:
a first delivery device, including a first catheter and a first handle;
a second delivery device, including a second catheter and a second handle; wherein the second catheter is extended through the first handle and the first catheter, and the second handle is positioned at a proximal side of the first handle; and
at least one limiting member removably coupled to the second catheter; wherein each of the at least one limiting members is coupled to a proximal end portion of the second catheter and is axially limited between the first handle and the second handle, so as to limit each of at least one axial displacement of the second handle relative to the first handle in a one-to-one correspondence, thereby controlling each of at least one axial displacement of the second catheter relative to the first catheter in a one-to-one correspondence.

In some embodiments of the present invention, at least one limiting member is removably coupled to the second catheter, so that each limiting member can be axially limited between the first handle and the second handle outside the human body, thereby limiting each axial displacement of the second handle relative to the first handle. Thus, under the navigation and monitoring of ultrasonic cardiogram formed by ultrasound, a user can confirm whether a corresponding limiting member needs to be removed according to the observed actual situation from the ultrasonic cardiogram, so as to respectively control each axial displacement of the second catheter relative to the first catheter in the human body, which ensures the reliability under ultrasound guidance, and reduce the dependence on ray guidance. Therefore, the medical delivery system provided in some embodiments of the present invention greatly protects the life and health of users and patients, and effectively improves the safety of interventional surgery.

### Brief Description of the Drawings

In order to illustrate technical solutions of embodiments of the present invention more clearly, hereinafter, accompanying drawings requiring to be used in the embodiments will be briefly introduced. Apparently, the accompanying drawings in the following description merely relate to some embodiments of the present invention, and for a person of ordinary skill in the art, other accompanying drawings can also be derived from these accompanying drawings without involving any inventive effort.
Fig. 1 illustrates a schematic structural diagram of a medical delivery system in some embodiments.
Figs. 2-6 illustrate schematic structural diagrams of a medical delivery system in some other embodiments.
Fig. 7 illustrates a schematic structural diagram of a medical delivery system including three limiting members in some embodiments.
Fig. 8 illustrates a schematic structural diagram of a medical delivery system including a dilator in some embodiments.
Fig. 9 illustrates a schematic assembly diagram of a first delivery device and a dilator which are mounted in place in some embodiments.
Fig. 10 illustrates a schematic diagram of a scenario in which the dilator of Fig. 9 is disengaged from the first delivery device.
Fig. 11 illustrates a schematic state diagram of delivering a first delivery device into a human body using a dilator.
Fig. 12 is a schematic structural diagram of a visible marker in Fig. 11.
Fig. 13 illustrates a schematic state diagram of mounting a third delivery device and a limiting member to a second delivery device.
Fig. 14 illustrates a schematic state diagram of delivering the mounted second delivery device of Fig. 13 into a human body along the first delivery device.
Fig. 15 illustrates a schematic state diagram of a medical delivery system with a first limiting member of Fig. 14 removed.
Fig. 16 illustrates a schematic state diagram of a medical delivery system with a second limiting member of Fig. 15 removed.
Figs. 17-18 illustrate schematic state diagrams of a medical delivery system including two limiting members in some other embodiments.
Figs. 19-20 illustrate schematic structural diagrams of a medical delivery system including a loader in some embodiments.
Fig. 21 illustrates a schematic structural diagram of a limiting member in some embodiments.

The following Detailed Description of the Embodiments will be used to further illustrate the present invention in combination with the accompanying drawings.

### Detailed Description of the Embodiments

Hereinafter, the technical solutions in the embodiments of the present invention will be described clearly and thoroughly with reference to the accompanying drawings of the embodiments of the present invention. Obviously, the embodiments as described are only some of the embodiments of the present invention, and are not all of the embodiments. On the basis of the embodiments in the present invention, all other embodiments obtained by a person of ordinary skill in the art without any inventive effort shall all fall within the scope of protection of the present invention.

In addition, the following illustration of embodiments is made with reference to the accompanying drawings, and is used to illustrate specific embodiments of the present invention which can be implemented. Directional terms, such as "upper", "lower", "front", "rear", "left", "right", "inner", "outer", "side surface", and the like, as referred to in the present invention, are only made with reference to the directions of the accompanying drawings. Therefore, the directional terms are used to better and more clearly explain and understand some embodiments of the present invention, rather than indicating or implying that an apparatus or an member referred to must have a specific orientation, and be constructed and operated in the specific orientation, and therefore said terms cannot be understood as limitation to some embodiments of the present invention.

It should be noted that, in order to more clearly describe a medical delivery system provided by some embodiments of the present invention, the terms "proximal end" and "distal end" defined in the description of some embodiments of the present invention are both customary terms in the field of interventional medical treatment. In particular, "distal end" denotes an end away from an operator during a surgical procedure, and "proximal end" denotes an end close to the operator during the surgical procedure; the direction of a rotation central axis of objects such as a cylinder body and a tube body is defined as an axial direction or a longitudinal axis; a circumferential direction is a direction around the axis (perpendicular to the axis, and also perpendicular to a cross-sectional radius) of the objects; and a radial direction is the direction along a diameter or radius. It is to be noted that the word "end" appearing in terms "proximal end", "distal end", "one end", "another end", "first end", "second end", "initial end", "tail end", "two ends", "free end", "upper end" and "lower end", etc. is not limited to an end head, end point or end face, but also includes a portion extending an axial distance and/or radial distance from the end head, end point or end face on the member to which the end head, end point or end face belongs. Unless otherwise defined, all technical and scientific terms used in some embodiments of the present invention have the same meanings as those commonly understood by a person skilled in the art to which the present invention belongs. The customary terms used in the description of some embodiments of the present invention are for the purpose of describing particular embodiments only, and cannot be understood as limiting some embodiments of the present invention.

Some embodiments of the present invention provide a medical delivery system 100, wherein the medical delivery system 100 enables intervention from extracorporeal access to intracorporeal tissue, such as heart tissues, and positions a distal end thereof at an expected location of the intracorporeal tissue, so as to implement interventional treatment under different interventional routes using an implantable apparatus 800 removably coupled to the medical delivery system 100, such as to implement intervention treatment via a transapical route or via a transcatheter route.

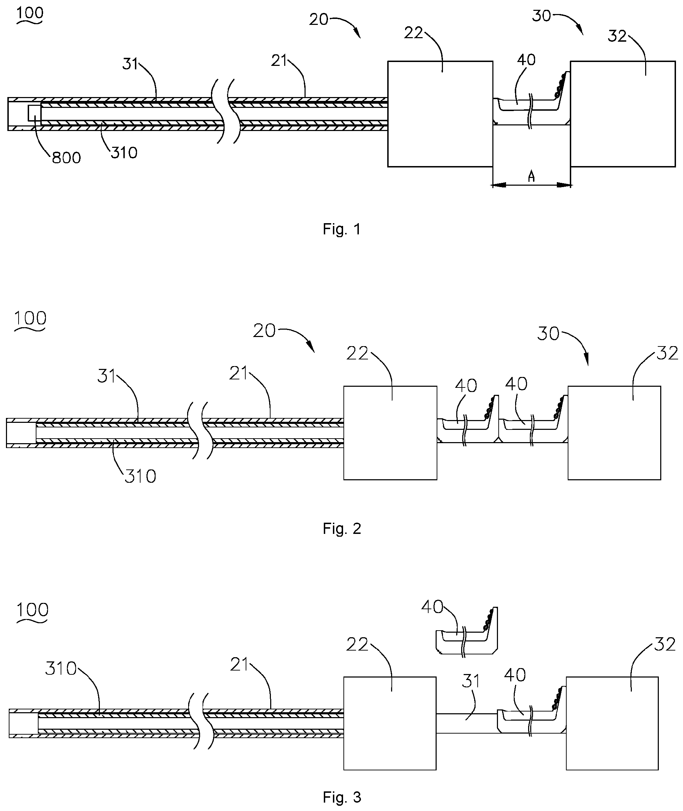

For some applications, as shown in Figs. 1-2, the medical delivery system 100 has a limiting function. The medical delivery system 100 includes a first delivery device 20, a second delivery device 30, and at least one limiting member 40, wherein the at least one limiting member 40 is configured to limit axial movement of the second delivery device 30 relative to the first delivery device 20.

Specifically, the first delivery device 20 has a first catheter 21 and a first handle 22, and the second delivery device 30 has a second catheter 31 and a second handle 32. The second catheter 31 can extend through the first delivery device 20, such as extend through the first handle 22 and the first catheter 21, and the second handle 32 can be positioned at a proximal side of the first handle 22.

In some embodiments, a proximal end of the first catheter 21 is coupled to the first handle 22, and a proximal end of the second catheter 31 is coupled to the second handle 32. The coupling mode above can include fixed coupling, for example, the first catheter 21 is fixed and immovable relative to the first handle 22, and the second catheter 31 is fixed and immovable relative to the second handle 32. Certainly, the coupling mode can also include active coupling, for example, the proximal end of the first catheter 21 is axially fixedly coupled into the first handle 22, but can twist, in particular can twist along a longitudinal axis of the first catheter 21; and the proximal end of the second catheter 31 is axially fixedly coupled into the second handle 32, but can twist, in particular can twist along a longitudinal axis of the second catheter 31.

Further, each of the at least one limiting member 40 is removably coupled to the second catheter 31. Each of the at least one limiting member 40 can be coupled to, for example, frictionally coupled to a proximal end portion of the second catheter 31, and is axially limited between the first handle 22 and the second handle 32, so as to limit each of at least one axial displacement of the second handle 32 relative to the first handle 22 in a one-to-one correspondence, thereby controlling each of at least one axial displacement of the second catheter 31 relative to the first catheter 21 in a one-to-one correspondence. Specifically, the axial displacement is an axial displacement of the second catheter 31 moving in the first catheter 21, or an axial displacement of the second catheter 31 moving out of the first catheter 21, or an axial displacement of an implantable apparatus 800 accommodated in or coupled to a distal end of the second catheter 31 moving out of the first catheter 21. Certainly, the axial displacement can also be another distance required in other application scenarios during an interventional procedure, and details will not be repeated herein again.

It can be understood that during the implementation of interventional procedures of the medical delivery system 100, most portion of the first catheter 21 and most portion of the second catheter 31 are located in a human body which are not visible by naked eyes. Thus, ultrasound guidance has irreplaceable navigation and monitoring functions in interventional procedures such as TEER surgery. Further, in order to reduce the use frequency and use time of Rays during an interventional procedure, even without using Rays and only relying on Ultrasound to complete the interventional procedure, in some embodiments of the present invention, at least one limiting member 40 is detachably connected to the second catheter 31. Each limiting member 40 can be axially limited between the first handle 22 and the second handle 32 outside the human body, so as to limit each axial displacement of the second handle 32 relative to the first handle 22. Thus, under the navigation and monitoring of ultrasonic cardiogram formed by Ultrasound, a user can confirm whether a corresponding limiting member 40 needs to be removed according to the observed actual situation from the ultrasonic cardiogram, so as to respectively control each axial displacement of the second catheter 31 relative to the first catheter 21 located in the human body.

In other words, the medical delivery system 100 in some embodiments of the present invention ensures reliability under ultrasound guidance, and reduces dependence on ray guidance, thereby greatly protecting the life and health of users and patients, and effectively improving the safety of interventional procedures.

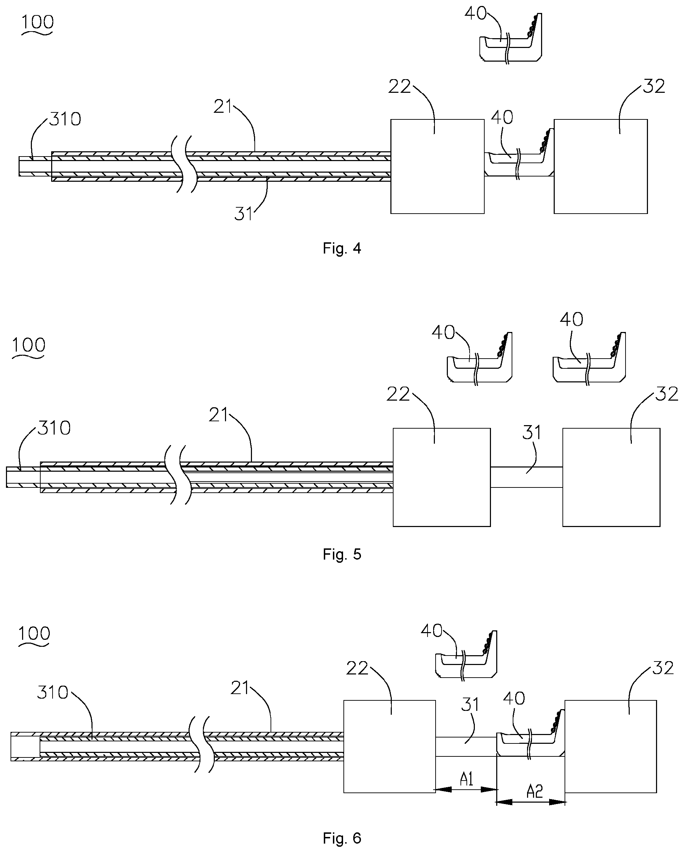

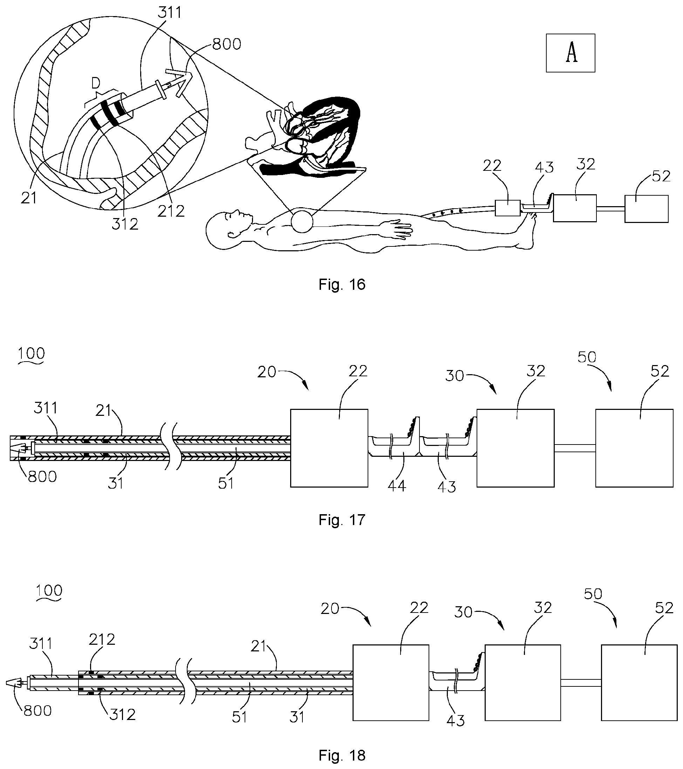

Further, each of the at least one limiting member 40 is removable from the second catheter 31, to release a corresponding axial displacement of the at least one axial displacement of the second handle 32 relative to the first handle 22 that is restricted. In some embodiments, as shown in Figs. 2-5, each limiting member 40 is removable from the second catheter 31, to release the corresponding constraint on axial displacement, thereby allowing a distal end portion 310 of the second catheter 31 to move from being completely accommodated in a distal end location in the first catheter 21 as shown in Fig. 2, to at least partially extending out of the first catheter 21 and positioned at an expected target location as shown in Figs. 4 and 5. In some embodiments, the expected target location can include a location at which the distal end portion 310 of the second catheter 31 is perpendicular to an annular plane of a heart valve as shown in Fig. 16, and can also include a location at which a first radiopaque unit 212 is continuously maintained between two second radiopaque units 312 as shown in Fig. 16. For details, reference is made to subsequent discussion of Fig. 16, and details will not be described herein again.

Certainly, in some other embodiments, please continue to refer to Figs. 2-5, there are at least two limiting members 40, the first handle 22, the at least two limiting members 40 and the second handle 32 are abutting axially in sequence from distally to proximally. The at least two limiting members 40 can be sequentially removed from the second catheter 31, for example, can be sequentially removed from the second catheter 31 from distally to proximally, or can be sequentially removed from the second catheter 31 from proximally to distally, so as to sequentially release the corresponding constraint on axial displacement; and then, the distal end portion 310 of the second catheter 31 is enable to move from being completely accommodated in the distal end location in the first catheter 21 as shown in Fig. 2, to at least partially extending out of the first catheter 21 and positioned at the expected target location as shown in Figs. 4 and 5.

Specifically, as shown in Fig. 2, when the two limiting members 40 axially abut against the first handle 22 and the second handle 32 sequentially, the distal end portion 310 of the second catheter 31 is completely accommodated in the distal end location in the first catheter 21. Then, one limiting member 40 is removed from the second catheter 31 from distally to proximally in the manner as shown in Fig. 3, and the second handle 32 is pushed distally in the manner as shown in Fig. 4 until the other limiting member 40 abuts between the first handle 22 and the second handle 32.

Given that the conditions of intracorporeal tissues vary within each patient, in some embodiments, as shown in Fig. 4, when the other limiting member 40 abuts between the first handle 22 and the second handle 32, after moving out of the first catheter 21, the distal end portion 310 of the second catheter 31 can reach the expected target location so as to be positioned at the target location. However, in some other embodiments, when the other limiting member 40 abuts between the first handle 22 and the second handle 32, it is observed from the ultrasonic cardiogram formed by ultrasound that the distal end portion 310 of the second catheter 31 can move out of the first catheter 21, but cannot reach the expected target location. In this situation, please refer to Fig. 5, a user needs to further remove the other limiting member 40 from the second catheter 31, and push the second handle 32 distally in conjunction with the ultrasonic cardiogram, so as to position the distal end portion 310 of the second catheter 31 at the expected target location.

It can be understood that each limiting member 40 can also play the role of notifying the user. Each limiting member 40 is used for providing a visual indication about the current state and the state at a next step of the distal end of the medical delivery system 100, so as to achieve the effects of notifying the user and attracting the user's attention.

In some embodiments, each limiting member 40 is connected to a fixed location on the second catheter 31. Specifically, as shown in Figs. 1 and 6, the proximal end portion of the second catheter 31 forms axial limiting segments, such as A in Fig. 1 or A1 and A2 in Fig. 6. Each limiting member 40 can be axially limited to a corresponding axial limiting segment, and the axial length of each axial limiting segment is equal to the corresponding axial displacement, specifically equal to the corresponding axial displacement of the second handle 32 relative to the first handle 22, and also equal to the corresponding axial displacement of the second catheter 31 relative to the first catheter 21.

For example, in Fig. 1, the limiting member 40 is connected to the axial limiting segment A of the second catheter 31, and is axially limited in the axial limiting segment A. In addition, in Fig. 6, two axially connected axial limiting segments, i.e. first axial limiting segment A1 and second axial limiting segment A2 are formed on the proximal end portion of the second catheter 31. A distal limiting member 40 is connected to the first axial limiting segment A1 of the second catheter 31, and is axially limited in the first axial limiting segment A1. A proximal limiting member 40 is connected to the second axial limiting segment A2 of the second catheter 31, and is axially limited in the second axial limiting segment A2. The distal limiting member 40 can be firstly removed from the second catheter 31. Then, the second catheter 31 can be axially moved with the proximal limiting member 40 , and if necessary, the proximal limiting member 40 is removed from the second catheter 31, and finally the distal end portion 310 of the second catheter 31 is positioned at the expected target location.

Hereinafter, a plurality of application scenarios of the medical delivery system 100 are described by taking the number of limiting members 40 being two or three and the medical delivery system 100 being used to implement transcatheter mitral valve clipping as an example. The implantable apparatus 800 is a valve clip, and the valve clip can treat mitral valve regurgitation by capturing an anterior leaflet and a posterior leaflet of the mitral valve and closing a gap between the anterior leaflet and posterior leaflet.

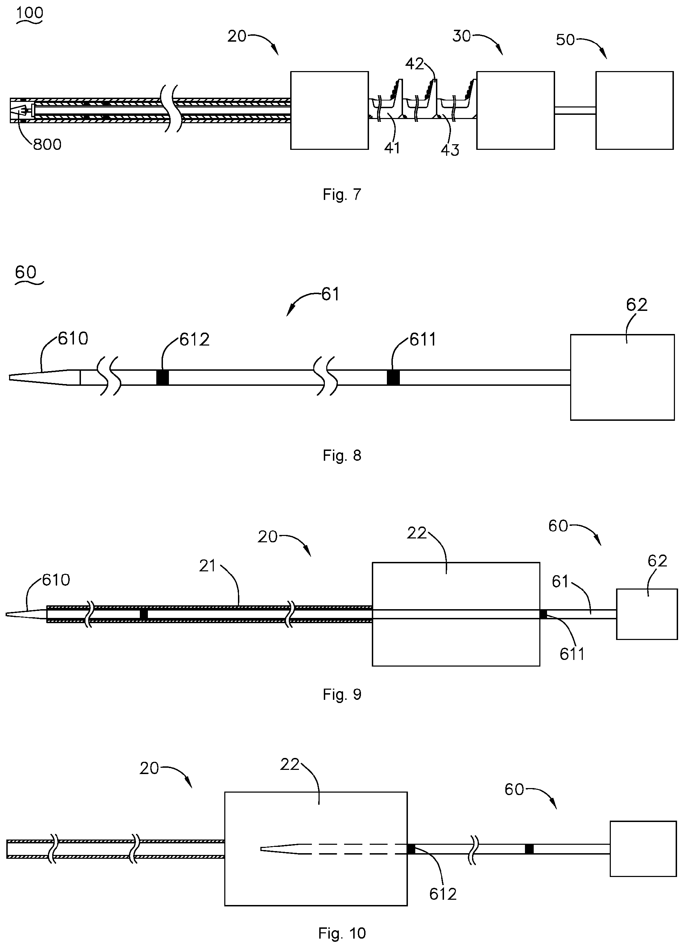

As shown in Fig. 7, for some applications, the medical delivery system 100 includes a first delivery device 20, a second delivery device 30, a third delivery device 50, a first limiting member 41, a second limiting member 42, and a third limiting member 43, wherein the implantable apparatus 800 is removably coupled to the third delivery device 50.

Then, during transfemoral venous puncture, after a route of femoral vein - postcava - right atrium - atrial septum - left atrium - mitral valve - left ventricle is established via a guide wire, the first delivery device 20 can be delivered into the human body along the route of the guide wire. Specifically, in order to ensure that the first delivery device 20 can smoothly pass through a puncture opening of the atrial septum from the right atrium of the human body's heart to the left atrium, as shown in Fig. 8, the medical delivery system 100 further includes a dilator 60, wherein the dilator 60 is used to communicate with an access route established by the guide wire to assist the first delivery device 20, such that the first delivery device 20 can smoothly pass through the puncture opening by dilating the puncture opening of the atrial septum.

Specifically, the dilator 60 includes a sheath 61 and an operating handle 62 fixedly coupled to a proximal end of the sheath 61, and the guide wire can sequentially extend axially through an inner lumen of the operating handle 62 and an inner lumen of the sheath 61. A variable-diameter portion 610 having a taper is formed at a distal end of the sheath 61, for effectively reducing the resistance when passing through the puncture opening of the atrial septum. In some embodiments, the axial length of the variable-diameter portion 610 is 25mm-30mm.

Generally, a correct mounting location of the dilator 60 and the first delivery device 20 is that the variable-diameter portion 610 of the dilator 60 completely or fully extends out of a distal end opening of the first catheter 21 of the first delivery device 20. In view of the long axial lengths of both the dilator 60 and the first delivery device 20 with transcatheter approach, it is necessary for the user to repeatedly confirm whether the mounting locations of the two are in place before the procedure, thereby severely affecting the efficiency of the procedure. Accordingly, in order to increase the efficiency of the procedure, in some embodiments, a first indicator 611 is provided on the sheath 61 of the dilator 60, and the first indicator 611 is provided on a proximal end portion of the sheath 61. Then, when needing to mount the dilator 60 to the first delivery device 20 correctly, the first indicator 611 can notify the user that the dilator 60 has entered and has been mounted to the correct location of the first delivery device 20. Specifically, as shown in Fig. 9, when the first indicator 611 is aligned with a proximal end surface of the first handle 22 of the first delivery device 20, the user is notified that the dilator 60 and the first delivery device 20 have been mounted in place.

Further, in order to prevent excessive force, too fast force application, or too large amplitude of action of the user in the process of withdrawing the dilator 60 from the first delivery device 20 from affecting excessive shaking of the first delivery device 20 which is still located in the human body, in some other embodiments, a second indicator 612 is further provided on the sheath 61 of the dilator 60, and the second indicator 612 is provided at a distal end portion of the sheath 61. For example, the second indicator 612 is provided at a distal end portion which is 3 cm - 15 cm from a distal end surface of the sheath 61. As shown in Fig. 10, when the second indicator 612 extends out of the proximal end surface of the first handle 22, it indicates that the dilator 60 is about to be withdrawn from the first delivery device 20, and the second indicator 612 is configured to prompt the user to slow down the disengagement speed of the dilator 60, so as to prevent the first delivery device 20 from excessive shaking, thereby improving the safety of the procedure.

The first indicator 611 and the second indicator 612 are one of tactile feedback, auditory feedback, and visual feedback. In this embodiment, the first indicator 611 and the second indicator 612 are two annular markers provided on an outer surface of the sheath 61, for example, color markers.

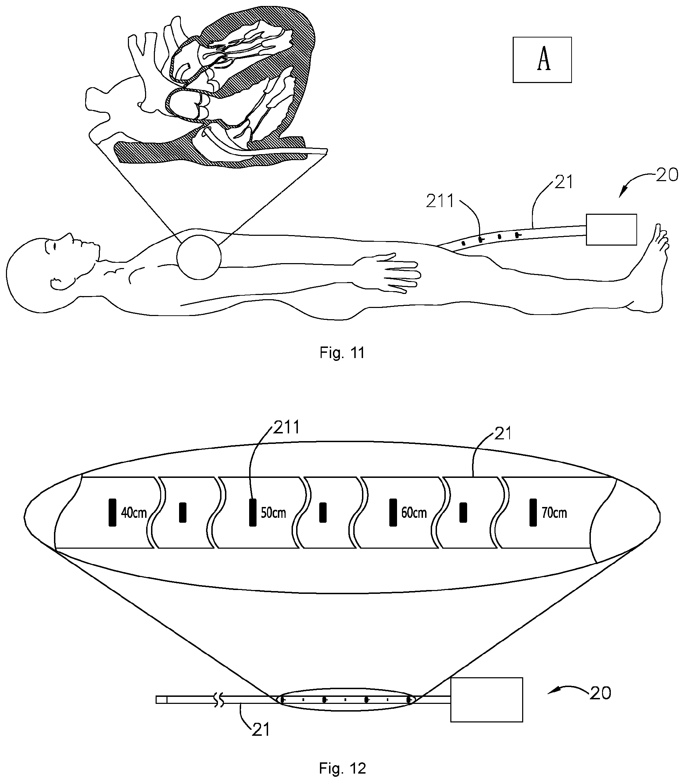

It can be understood that the first delivery device 20 is used to establish a route from outside of the patient's body to the left atrium, and to position a distal end of itself to a target location of an intracorporeal tissue, thereby providing a route for delivering the implantable apparatus 800 implementing treatment to a treatment area, so as to achieve interventional treatment. Then, please refer to Fig. 11, when the first delivery device 20 and the dilator 60 which have been correctly mounted are delivered into the human body along the guide wire, the delivery process can confirm whether the distal end of the first delivery device 20 has reached near the atrial septum via an ultrasonic cardiogram formed by an ultrasound device A and a plurality of visible markers 211 on the first delivery device 20. Once completion of establishment of the access route, the guide wire and the dilator 60 are withdrawn.

In some embodiments, as shown in Fig. 12, the plurality of visible markers 211 are axially spaced apart from each other on an outer surface of the first catheter 21 of the first delivery device 20, so as to visualize the length of the first catheter 21 entering the human body. The plurality of visible markers 211 are disposed at equal intervals. For example, the plurality of visible markers 211 are scale markers which are coated on, sprayed on, etched on, or printed on the first catheter 21 and used for displaying the length, and each scale marker displays a distance between the scale marker and a distal end surface of the first catheter 21. The plurality of scale markers include numbers for displaying length, and the numbers gradually increase from distally to proximally at equal intervals, thereby ensuring that the user can view the length of the first catheter 21 entering the human body by means of the scale markers.

In some embodiments, the number of the visible markers 211 is 7 - 15, and in order to observe the visible markers 211 from multiple directions, the plurality of visible markers 211 are disposed as 1 - 4 groups in the circumferential direction of the first catheter 21. Of course, the plurality of visible markers 211 can also be circumferential ring-shaped scales extending along the circumferential direction of the first catheter 21. Also in some embodiments, the visible marker 211 at the most distal end is provided within a range of 0 - 40 cm from the distal end surface of the first catheter 21, and the range of a spacing between every two adjacent visible markers 211 is 5 cm - 10 cm.

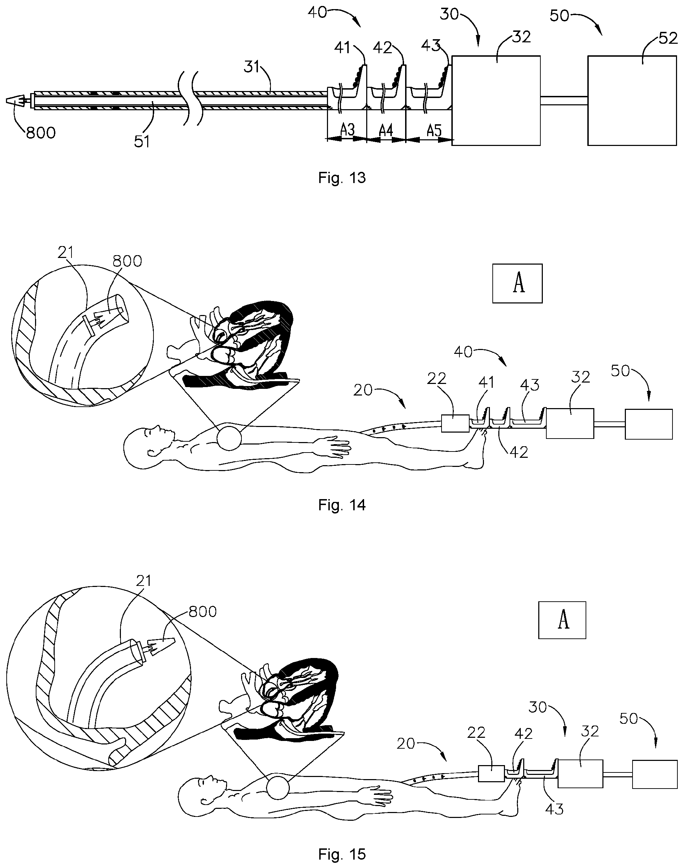

Please refer to both Fig. 13 and Fig. 14, in this case, as shown in Fig. 13, the second delivery device 30 and the third delivery device 50 have been assembled. Specifically, the third delivery device 50 has a third catheter 51 and a third handle 52, and a proximal end of the third catheter 51 is coupled to the third handle 52, such as fixedly coupled. The third catheter 51 can extend through the second delivery device 30, such as extend through the second handle 32 and the second catheter 31, and the third handle 52 can be positioned at a proximal side of the second handle 32.

Moreover, the implantable apparatus 800 is removably coupled to a distal end of the third catheter 51. For a larger-sized implantable apparatus 800, such as a valve clip, it is generally positioned outside the distal end of the second catheter 31 of the second delivery device 30; in contrast, for a smaller-sized implantable apparatus 800, such as a heart valve, it is generally positioned and accommodated within the inner lumen of the distal end of the second catheter 31 of the second delivery device 30.

Then, the first limiting member 41, the second limiting member 42 and the third limiting member 43 are coupled to a proximal end portion of the second catheter 31, and a proximal end of the third limiting member 43 abuts against a distal end surface of the second handle 32. The first limiting member 41, the second limiting member 42 and the third limiting member 43 are axially connected to the second catheter 31 end to end in the direction from distally to proximally, so as to form axial limiting of the first limiting member 41, the second limiting member 42 and the third limiting member 43. For example, three axially connected axial limiting segments, i.e. a third axial limiting segment A3, a fourth axial limiting segment A4 and a fifth axial limiting segment A5 are formed at the proximal end portion of the second catheter 31. The first limiting member 41 is connected to the third axial limiting segment A3 of the second catheter 31, and is axially limited in the third axial limiting segment A3. The second limiting member 42 is connected to the fourth axial limiting segment A4 of the second catheter 31, and is axially limited in the fourth axial limiting segment A4. The third limiting member 43 is connected to the fifth axial limiting segment A5 of the second catheter 31, and is axially limited in the fifth axial limiting segment A5. In some embodiments, the first limiting member 41 has an axial length equal to that of the third axial limiting segment A3, the second limiting member 42 has an axial length equal to that of the fourth axial limiting segment A4, and the third limiting member 43 has an axial length equal to that of the fifth axial limiting segment A5.

Next, according to the method as shown in Fig. 14, the second delivery device 30 and the third delivery device 50 provided with the limiting members 40 are slowly delivered into the first delivery device 20, until the distal end of the first limiting member 41 abuts against the proximal end surface of the first handle 22 of the first delivery device 20. At this time, the first handle 22, the first limiting member 41, the second limiting member 42, the third limiting member 43 and the second handle 32 are abutting against each other axially in sequence, so as to notify the user that the implantable apparatus 800 has been located in a distal end lumen of the first catheter 21 and is about to extend out of the distal end lumen of the first catheter 21 to enter into the left atrium.

The delivery process is achieved by axial limiting of the first limiting member 41, the second limiting member 42 and the third limiting member 43, and can also cooperate with the ultrasonic cardiogram formed by the ultrasonic device A if necessary. In this case, the configuration of the limiting members 40 is used for prompting the user that the first limiting member 41 should be removed, so as to allow the second delivery device 30 and the third delivery device 50 to continue to be pushed, and is used for prompting the user that the delivery speed needs to be slowed down in subsequent operation steps, so as to prevent the implantable apparatus 800 from accidentally touching the atrial wall of the human body.

Further, the first limiting member 41, the second limiting member 42, and the third limiting member 43 are used to be removed from the second catheter 31 from distally to proximally in sequence, so as to move the implantable apparatus 800 from a location in which the implantable apparatus 800 is substantially completely accommodated in the first catheter 21 as shown in Fig. 14 to a location in which the implantable apparatus 800 completely extends out of the first catheter 21 as shown in Fig. 16. Meanwhile, in the scenario as shown in Fig. 16, the distal end portion 310 of the second catheter 31 is positioned at the expected target location, such as a target location where the distal end portion 310 of the second catheter 31 is perpendicular to the annular plane of the heart valve and has reached a straddling zone D.

Specifically, when the first limiting member 41 is removed as shown in Fig. 15, the user should slowly and continuously push the second delivery device 30 and the third delivery device 50, until the distal end of the second limiting member 42 abuts against the proximal end surface of the first handle 22 of the first delivery device 20. In this case, the first handle 22, the second limiting member 42, the first limiting member 41 and the second handle 32 are abutting against each other axially in sequence, so as to notify the user that the implantable apparatus 800 has completely extended from the first catheter 21 and has been completely positioned within the left atrium, and also to prompt the user to slow down the delivery speed in subsequent operation steps, and also to make use of the ultrasonic cardiogram formed by the ultrasonic device A, to avoid the risk of the implantable apparatus 800 accidentally touching the atrial wall of the human body during further extension. Of course, the user can further adjust the orientation of the first catheter 21 by using the ultrasonic cardiogram formed by the ultrasonic device A according to the actual situation of the mitral valve of each patient.

Necessarily, in order to ensure that the implantable apparatus 800 can completely extend out from the first catheter 21 after the first limiting member 41 is removed, in some embodiments, the axial length of the first limiting member 41 is greater than or equal to the axial length of the implantable apparatus 800, or greater than or equal to the axial distance between a distal end surface of the implantable apparatus 800 and a distal end surface of the second catheter 31. In some embodiments, the range of the axial length of the first limiting member 41 is 18-25 mm.

Next, when the second limiting member 42 is removed as shown in Fig. 16, the user should slowly and continuously push the second delivery device 30 and the third delivery device 50, until the distal end of the third limiting member 43 abuts against the proximal end surface of the first handle 22 of the first delivery device 20. At this time, the first handle 22, the third limiting member 43 and the second handle 32 can axially abut sequentially, so as to notify the user that the distal end portion 310 of the second catheter 31 has reached the straddling zone D. Specifically, the distal end portion of the first catheter 21 includes a first radiopaque unit 212, and the distal end portion 310 of the second catheter 31 includes two second radiopaque units 312 axially spaced apart. When the first radiopaque unit 212 of the first catheter 21 enters into a region between the two second radiopaque units 312 of the second catheter 31, it is indicated that the distal end portion 310 of the second catheter 31 reaches the straddling zone D, that is, the expected target location is reached, and the expected target location includes the location where the first radiopaque unit 212 is continuously maintained between the two second radiopaque units 312.

In some embodiments, the first radiopaque unit 212 and the second radiopaque units 312 are developing bands fixed in the first catheter 21 or the second catheter 31, or are developing markers coated, sprayed or printed on the first catheter 21 or the second catheter 31.

In view of the fact that the second catheter 31 needs to be kept perpendicular to the annular plane as far as possible after extending out from the distal end of the first catheter 21, in the present invention, both the first catheter 21 and the second catheter 31 are set as bending-adjustable sheaths, so as to better and more quickly adjust the distal end of the second catheter 31 to an angle perpendicular to the annular plane under the cooperation of the first and second catheters. Specifically, the first catheter 21 has a bending-adjustable segment in the distal end, and the distal end portion 310 of the second catheter 31 further includes a deflectable distal end segment 311. The implantable apparatus 800 is positioned outside the distal end segment 311, the first limiting member 41 is configured to limit the implantable apparatus 800 to completely extend out from the first catheter 21, and the second limiting member 42 is configured to limit the distal end segment 311 to completely extend out from the first catheter 21. Of course, in other embodiments, under different intervention routes, only one of the first catheter 21 and the second catheter 31 is configured as a bending-adjustable sheath. The design of the bending-adjustable or deflectable structure belongs to the related art, and will not be described herein again.

In view of the fact that the straddling zone D of the second catheter 31 is located at the proximal end of the distal end segment 311, necessarily, in order to ensure that the distal end segment 311 of the second catheter 31 can completely extend out from the first catheter 21 after the removal of the second limiting member 42, so as to ensure that the second catheter 31 can smoothly reach the straddling zone D, in some embodiments, the axial length of the second limiting member 42 is greater than or equal to the axial length of the distal end segment 311. In some embodiments, the axial length of the second limiting member 42 can range from 18 mm to 25 mm.

Then, the user can adjust the orientation of the second catheter 31 by means of the ultrasonic cardiogram formed by the ultrasound device A, while the third limiting member 43 will always remain in the second catheter 31. Given that the second radiopaque units 312 of the second catheter 31 are provided at a proximal end of the distal end segment 311, the third limiting member 43 serves to continuously abut between the first handle 22 and the second handle 32, so as to force the first radiopaque unit 212 to continuously maintain between the two second radiopaque units 312, until the procedure ends, so as to prevent the second catheter 31 from extending out too much due to misoperation during the procedure and from touching the atrial wall of the human body, thereby improving the safety of the procedure. That is, the third limiting member 43 can limit the distal ends of the first delivery device 20 and the second delivery device 30 in the target location of the intracorporeal tissue, respectively, and axial locations of the first and second delivery devices keep immovable, so as to stably ensure that the implantable apparatus 800 at the distal end of the third delivery device 50 implements safe interventional treatment on the intracorporeal tissue.

Of course, in some embodiments, when the distal end of the third limiting member 43 abuts against the proximal end surface of the first handle 22 of the first delivery device 20, it is observed from the ultrasonic cardiogram formed by the ultrasound device A that the second catheter 31 has not reached the straddling zone D, so the user can further remove the third limiting member 43 to ensure that the second catheter 31 can reach the straddling zone D.

Then the distal end of the third delivery device 50 is positioned and arrives at the target location by co-adjustment of the first delivery device 20, the second delivery device 30, and the third delivery device 50. When the implantable apparatus 800 which is specifically a valve clip captures and clips the anterior leaflet and the posterior leaflet of the mitral valve safely and effectively, the gap between the anterior leaflet and the posterior leaflet is reduced or disappeared during the systolic phase of the heart, and the orifice of the mitral valve becomes double orifices during the diastolic phase of the heart, thus proving that the effect of reducing or eliminating mitral valve regurgitation is achieved. Next, a disengagement mechanism (not shown in the figures) at the end of the third handle 52 is manipulated, to disengage the implantable apparatus 800 from the distal end of the third catheter 51 to release the implantable apparatus 800. Finally, the medical delivery system 100 is withdrawn, completing the procedure. At this time, the implantable apparatus 800 will remain in the human body for a long period of time, thereby continuously preventing the mitral valve regurgitation from occurring.

For some other applications, please refer to Figs. 17-18, the medical delivery system 100 includes a first delivery device 20, a second delivery device 30, a third delivery device 50, an implantable apparatus 800, and a third limiting member 43 and a fourth limiting member 44. After the medical delivery system 100 shown in Fig. 17 is delivered into the human body in the method as shown in Fig. 14, the first handle 22, the fourth limiting member 44, the third limiting member 43 and the second handle 32 are abutting axially in sequence.

The fourth limiting member 44 and the third limiting member 43 are used for being removed from the second catheter 31 sequentially from distally to proximally. Thus, when the fourth limiting member 44 is removed from the second catheter 31, the second handle 32 can move axially relative to the first handle 22, to synchronously allow the implantable apparatus 800 and the distal end segment 311 of the second catheter 31 to completely extend out of the first catheter 21. In addition, the third limiting member 43 can continuously abut between the first handle 22 and the second handle 32, so as to force the first radiopaque unit 212 to continuously maintain between the two second radiopaque units 312. It can be understood that in view of the fact that except that the number of limiting members 40 is different, structures of the other components of the medical delivery system 100 shown in Figs. 17-18 and the medical delivery system 100 shown in Figs. 7-16 are the same, details will not be repeated herein again.

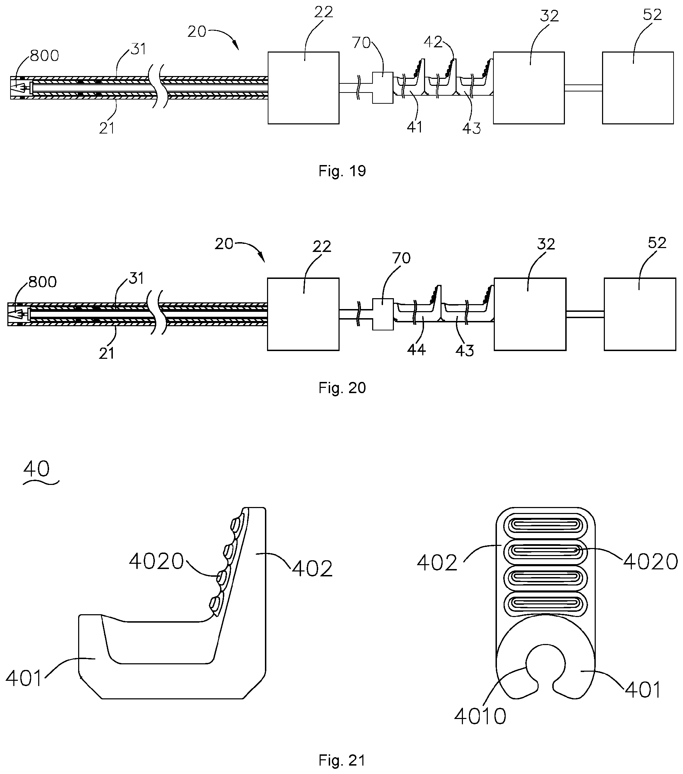

It can be understood that for a larger-sized implantable apparatus 800, such as a valve clip, in order to ensure that the implantable apparatus can be smoothly inserted into the first delivery device 20, the medical delivery system 100 further includes a loader 70. Specifically, please refer to Figs. 19-20, a distal end of the loader 70 is mounted within the first handle 22 of the first delivery device 20, to guide the implantable apparatus 800 positioned outside the second catheter 31 into the first handle 22 and through the first catheter 21, so that the larger-sized implantable apparatus 800 can be smoothly loaded into the first delivery device 20. In this case, in the embodiment as shown in Fig. 19, the first limiting member 41, the second limiting member 42 and the third limiting member 43 can axially abut between the loader 70 and the second handle 32 sequentially. In the embodiment as shown in Fig. 20, the fourth limiting member 44 and the third limiting member 43 axially abut between the loader 70 and the second handle 32 sequentially.

In addition, with regard to the structure of the limiting member 40, please refer to Fig. 21, the limiting member 40 includes an engagement portion 401, wherein an axially extending inner engagement surface 4010 is formed on the engagement portion 401, and the inner engagement surface 4010 is configured to frictionally coupled to an outer surface of the second catheter 31, so as to achieve axial limiting of the two. For example, the inner engagement surface 4010 is frictionally sleeved over the outer surface of the second catheter 31, so that stable axial limiting is achieved between the inner engagement surface 4010 and the outer surface of the second catheter 31 by using a friction force between the two.

In some embodiments, the engagement portion 401 of the limiting member 40 has elasticity, and the cross section of the inner engagement surface 4010 is configured a C-shaped configuration having an opening. Regarding the limiting member 40, by making the opening of the C-shaped configuration abut against the second catheter 31, and by applying an action force to force the opening to dilate, the second catheter 31 is frictionally abutted against an inner cavity of the C-shaped configuration. The C-shaped configuration has two attributes, i.e. a firmly fixing attribute and a convenient detachment attribute, so as to ensure that the limiting member 40 can be firmly fixed on the outer surface of the second catheter 31 to avoid falling due to vibration, and can also be quickly detached from the second catheter 31 at different procedure stages.

Of course, in order to further ensure that the limiting member 40 can be stably limited at the second catheter 31, and can also be conveniently and quickly removed from the second catheter 31, in some embodiments, the axial length of the limiting member 40 is greater than or equal to the axial length of the corresponding inner engagement surface 4010. At this time, the contact area between the inner engagement surface 4010 and the second catheter 31 is necessarily reduced, thereby effectively ensuring quick removal of the limiting member 40.

In addition, in order to facilitate the user's manipulation and holding of the limiting member 40, the limiting member 40 further includes a holding portion 402, and the holding portion 402 is extended from the engagement portion 401 in a direction away from the opening. Of course, a high friction surface 4020, such as a surface having a plurality of protrusions or grooves, can also be provided on the holding portion 402, so as to increase the friction force of the holding portion 402.

It can be understood that the medical delivery system 100 can be applied to mitral valve repair, and of course, can also be applied to other heart interventional treatment medical instruments such as tricuspid valve repair and aortic repair. Thus, in order to adapt to different application scenarios, all technical features discussed in the medical delivery system 100 is used in combination with each other, which is not repeated herein again.

The content above relates to embodiments of the present invention. It should be noted that for a person of ordinary skill in the art, several improvements and modifications can also be made without departing from the principle of the present invention, and these improvements and modifications shall also be considered as within the scope of protection of the present invention.

## Claims

1. A medical delivery system having a limiting function, comprising:
a first delivery device, comprising a first catheter and a first handle;
a second delivery device, comprising a second catheter and a second handle; wherein the second catheter is extended through the first handle and the first catheter, and the second handle is positioned at a proximal side of the first handle; and
at least one limiting member removably coupled to the second catheter; wherein each of the at least one limiting member is coupled to a proximal end portion of the second catheter and is axially limited between the first handle and the second handle, so as to limit each of at least one axial displacement of the second handle relative to the first handle in a one-to-one correspondence, thereby controlling each of at least one axial displacement of the second catheter relative to the first catheter in a one-to-one correspondence.

2. The medical delivery system according to claim 1, wherein each of the at least one limiting member is removable from the second catheter, to release a corresponding axial displacement of the at least one axial displacement of the second handle relative to the first handle that is restricted, thereby allowing a distal end portion of the second catheter to move from being completely accommodated in a distal end location in the first catheter to at least partially extending out of the first catheter to position at an expected target location.

3. The medical delivery system according to claim 2, wherein a distal end portion of the first catheter comprises a first radiopaque unit, and the distal end portion of the second catheter comprises two second radiopaque units axially spaced apart, and the expected target location comprises a location where the first radiopaque unit is continuously maintained between the two second radiopaque units.

4. The medical delivery system according to claim 1, wherein at least one axial limiting segment is formed at a proximal end portion of the second catheter, each of the at least one limiting member is axially limited to a corresponding axial limiting segment of the at least one axial limiting segment, and each of the at least one axial limiting segment has an axial length equal to the corresponding axial displacement of the at least one axial displacement of the second catheter relative to the first catheter.

5. The medical delivery system according to any one of claims 1-4, wherein there are at least two limiting members, and the first handle, the at least two limiting members and the second handle are abutting axially in sequence from distally to proximally; and the at least two limiting members are sequentially removed from the second catheter, so as to sequentially release at least two corresponding axial displacements of the second handle relative to the first handle that are restricted.

6. The medical delivery system according to claim 5, wherein the medical delivery system further comprises a third delivery device, the third delivery device comprises a third catheter and a third handle, wherein an implantable apparatus is removably coupled to a distal end of the third catheter; wherein the third catheter is extended through the second handle and the second catheter, and the third handle is positioned at a proximal side of the second handle; and
the at least two limiting members comprise a first limiting member, a second limiting member and a third limiting member; the first handle, the first limiting member, the second limiting member, the third limiting member and the second handle are abutting against each other axially in sequence; and the first limiting member, the second limiting member and the third limiting member are configured to be removed from the second catheter from distally to proximally in sequence.

7. The medical delivery system according to claim 6, wherein the distal end portion of the second catheter comprises a deflectable distal end segment, and the implantable apparatus is positioned outside of the distal end segment; when the first limiting member is removed from the second catheter, the implantable apparatus can move to be completely extended out of the first catheter; and when the second limiting member is removed from the second catheter, the distal end segment can move to be completely extended out of the first catheter.

8. The medical delivery system according to claim 7, wherein an axial length of the first limiting member is greater than or equal to an axial distance from a distal end surface of the implantable apparatus to a distal end surface of the second catheter, or an axial length of the first limiting member is greater than or equal to an axial length of the implantable apparatus; and
an axial length of the second limiting member is greater than or equal to an axial length of the distal end segment.

9. The medical delivery system according to claim 7, wherein the distal end portion of the first catheter comprises a first radiopaque unit, and the distal end portion of the second catheter comprises two second radiopaque units axially spaced apart, and the second radiopaque units are disposed adjacent to a proximal side of the distal end segment; and the third limiting member is continuously abutting between the first handle and the second handle, so as to force the first radiopaque unit to continuously maintain between the two second radiopaque units.

10. The medical delivery system according to claim 5, wherein the medical delivery system further comprises a third delivery device, the third delivery device comprises a third catheter and a third handle, wherein an implantable apparatus is removably coupled to a distal end of the third catheter; wherein the third catheter is extended through the second handle and the second catheter, and the third handle is positioned at a proximal side of the second handle; and
the at least two limiting members comprise a third limiting member and a fourth limiting member; the first handle, the fourth limiting member, the third limiting member, and the second handle are abutting against each other axially in sequence; and the fourth limiting member and the third limiting member are configured to be removed from the second catheter from distally to proximally in sequence.

11. The medical delivery system according to claim 10, wherein the distal end portion of the first catheter comprises a first radiopaque unit, and the distal end portion of the second catheter comprises a deflectable distal end segment, and two second radiopaque units axially spaced apart, the two second radiopaque units are disposed adjacent to a proximal side of the distal end segment; and when the fourth limiting member is removed from the second catheter, the implantable apparatus and the distal end segment both can move to be completely extend out of the first catheter; and the third limiting member is continuously abutting between the first handle and the second handle, so as to force the first radiopaque unit to continuously maintain between the two second radiopaque units.

12. The medical delivery system according to any one of claims 6-11, wherein the medical delivery system further comprises a loader, wherein a distal end of the loader is mounted in the first handle so as to guide the implantable apparatus located outside the second catheter to enter into the first handle and to extend through the first catheter;
the first limiting member, the second limiting member and the third limiting member axially abut between the loader and the second handle in sequence; or the fourth limiting member and the third limiting member axially abut between the loader and the second handle in sequence.

13. The medical delivery system according to any one of claims 1-12, wherein each of the at least one limiting member comprises an engagement portion, wherein an axially extending inner engagement surface is formed on the engagement portion, and the inner engagement surface is configured to frictionally connected to an outer surface of the second catheter.

14. The medical delivery system according to claim 13, wherein the engagement portion has elasticity, and a cross section of the inner engagement surface is configured a C-shaped configuration having an opening.

15. The medical delivery system according to any one of claims 1-12, wherein
an outer surface of the first catheter is provided with a plurality of visible markers disposed at intervals along an axial direction, so as to visualize a length of the first catheter entering a human body; and/or,
the medical delivery system further comprises a dilator, wherein a sheath of the dilator is provided with a first indicator and a second indicator, the first indicator is disposed at a proximal end portion of the sheath, and the second indicator is disposed at a distal end portion of the sheath; and when the first indicator is aligned with a proximal end surface of the first handle, a user is notified that the dilator and the first delivery device have been mounted in place, and when the second indicator extends beyond the proximal end surface of the first handle, the user is prompted to slow down a disengagement speed of the dilator.
